(19) Europäisches Patentamt / European Patent Office / Office européen des brevets

(11) **EP 2 679 147 B1**

(12) **EUROPÄISCHE PATENTSCHRIFT**

(45) Veröffentlichungstag und Bekanntmachung des Hinweises auf die Patenterteilung:
**17.04.2019 Patentblatt 2019/16**

(21) Anmeldenummer: **13174043.3**

(22) Anmeldetag: **27.06.2013**

(51) Int Cl.:
*G06F 3/01* (2006.01)        *A61B 3/113* (2006.01)
*G06K 9/00* (2006.01)        *G02B 27/00* (2006.01)
*G06K 19/06* (2006.01)

(54) **Verfahren und Vorrichtung zur Kodierung von Augen- und Blickverlaufsdaten**

Method and device for encoding eye and viewing direction data

Procédé et dispositif de codage de données de suivi de l'oeil et du regard

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.06.2012 DE 102012105664**
**28.06.2012 US 201261665331 P**

(43) Veröffentlichungstag der Anmeldung:
**01.01.2014 Patentblatt 2014/01**

(73) Patentinhaber: **Hein, Oliver**
**20249 Hamburg (DE)**

(72) Erfinder: **Hein, Oliver**
**20249 Hamburg (DE)**

(74) Vertreter: **2K Patentanwälte Blasberg Kewitz & Reichel**
**Partnerschaft mbB**
**Schumannstrasse 27**
**60325 Frankfurt am Main (DE)**

(56) Entgegenhaltungen:
**WO-A1-2009/153724        US-A1- 2006 189 886**
**US-A1- 2009 086 165        US-A1- 2010 118 030**
**US-B2- 7 029 121**

**Beschreibung**

Die Erfindung betrifft ein Verfahren und eine Vorrichtung zur Kodierung von Augen- und Blickverlaufsdaten. Insbesondere betrifft die Erfindung eine Kodierung von Augen- und Blickverlaufsdaten für eine Blicksteuerung einer Mensch-Maschine-Interaktion. Die mittels der Erfindung durchgeführte Blicksteuerung soll beispielsweise im Bereich von Benutzer-abhängigen Augen- und Blickverlaufsgesteuerten Prozessen in technischen Vorrichtungen, wie z.B. Maschinen, Geräten, Computern, Anlagen, Fahrzeugen usw. eingesetzt werden.

Aus dem Stand der Technik sind an sich Verfahren und Vorrichtungen bekannt, die Augen- und Blickverlaufsdaten von Personen erfassen, um den sogenannten Blickpfad darzustellen, d.h. den zeitlichen Blickverlauf, welcher das Blickverhalten der Person beim Betrachten einer bildlichen Darstellung (z.B. Foto, Werbeplakat) oder einer Szene (z.B. Situation im Straßenverkehr, Film) wiedergibt. Üblicherweise werden Augen- und Blickverlaufsdaten mit Hilfe einer Blickerfassungsvorrichtung, eines sogenannten Eye-Tracker, erfasst.

Ein solches Verfahren und eine solche Blickerfassungsvorrichtung werden beispielsweise in der WO 99 / 18842 A1 beschrieben. Dort wird auch eine Datenauswertung beschrieben, mit der typische Augenbewegungsmuster erkannt werden können.

Ein weiteres Verfahren der bekannten Art wird z.B. in der US RE 40014 E1 offenbart.

Die WO 2009/153724 A1 offenbart ein Verfahren zur Abgabe von Steuerbefehlen mittels Augenbewegung, wobei das Bild einer Pupille erkannt wird und darin eine Anfangs-Fixierung des Pupillen-Mittelpunkts bezogen auf ein Referenzsystem. Dann wird das Zentrum eines Bündels gerader Linien, die vorbestimmte Richtungen aufweisen, an dem Pupillen-Mittelpunkt positioniert, wobei eine Ebene des Bildes in diskrete Bereiche unterteilt wird. Dann wird das Zentrum eines Bündels von Strahlen an dem Pupillen-Mittelpunkt positioniert, wobei das Strahlen-Bündel vorbestimmte Richtungen und Bedeutungen aufweist. Dann wird jeder diskrete Bereich in Zusammenhang gebracht mit einem bestimmten gerichteten Strahl, der zu dem Strahlen-Bündel gehört. Es wird die finale Position der Fixierung erkannt unter der Annahme des Pupillen-Mittelpunkts nach einer Verschiebung in Bezug auf die Anfangs-Fixierungs-Position. Es wird ein erster Verschiebungsvektor des Pupillen-Mittelpunkts identifiziert, der den Ursprung in der Anfangsposition des Pupillen-Mittelpunkts hat, und es wird ein Eckpunkt in der finalen Position des Pupillen-Mittelpunkts identifiziert. Es wird einer der ausgerichteten Strahlen aus dem Bündel mit der Richtung und Bedeutung eines ersten Verschiebungsvektors in Zusammenhang gebracht, der einem zu bewegenden Objekt zuordnenbar ist. Es wird dem ersten Verschiebungsvektor des Objektes ein Betrag zugemessen, der identisch mit einem Betrag des ersten Verschiebungsvektor des Pupillen-Mittelpunkts multipliziert mit einem vorbestimmten Verstärkungsfaktor ist.

In der US 2009 / 086165 A1 werden ein System und ein Verfahren zum adaptiven Festlegen von Fixierungs-Schwellenwerten für Augen-Betrachtungs-Verfolgungs-Daten und zum Identifizieren von Fixierungen in den Augen-Betrachtungs-Verfolgungs-Daten offenbart. Es werden Augen-Betrachtungs- Verfolgungs-Daten gesammelt. Ein Sakkaden-Schätzung kann berechnet werden durch Einsatz einer Überschreitungs-Wahrscheinlichkeit der Änderungen der Augen-Betrachtungs-Position. Fixierungen können durch Vergleich der Sakkaden-Schätzung mit Änderungen der Augen-Betrachtungs-Positionen über ein Zeitfenster bestimmt werden.

Die US 7 029 121 B2 offenbart Techniken zum vereinfachten Verwendung von Augen-Verfolgungs-Daten. Dabei können individuelle Augen-Verfolgungs-Daten zum Bestimmen verwendet werden, ob ein Individuum gegenwärtig auf einen bestimmten Bereich eines Sehfeldes geblickt hat. Eine Aggregation von Daten, die zu einer Vielzahl von Individuen korrespondiert, kann Trends und andere Daten liefern, die nützlich für Designer von grafischen Darstellungen (z.B. Webseiten, Werbung) und anderen sichtbaren Einzelheiten sind. Es können Darstellungen von aggregierten Bildbetrachtungsdaten, die beides angeben, betrachtete Bereiche wie auch nicht-betrachtete Bereiche, gewonnen werden durch Einsatz mehrerer verschiedener Techniken. Beispielsweise kann ein Prozentsatz von Betrachtern, die einen bestimmten Bereich betrachtet haben, als eine bestimmte Farbe dargestellt werden. Oder die betrachteten Bezugsbilder können verwischt werden auf der Basis eines Schärfegradienten und der Anzahl an Individuen, die verschiedene Bereiche betrachten. Die verschiedenen Bereiche, die wie betrachtet dargestellt werden, werden auf der Basis von Arten von Betrachtungs-Aktivitäten (z.B. Lesen, Betrachten) ausgewertet und werden mit bestimmten Bereichen assoziiert.

US 2010 / 118030 A1 offenbart Techniken zum Anzeigen von Pfad-bezogenen Informationen. Es werden Techniken zur Erzeugung und Anzeige von grafischen Darstellungen eines Pfades bereit gestellt. In einem Ausführungsbeispiel wird eine Radial-Vektor-Darstellung erzeugt und für einen Pfad ausgegeben. Die angezeigten Pfad-bezogenen Informationen werden dann analysiert und miteinander verglichen.

US 2006 / 189886 A1 offenbart ein System und ein Verfahren zur Quantifizierung und Abbildung visueller Vorsprünge zu einem visuellen Stimulus. Das System umfasst einen Prozessor; Software zum Empfang von Daten, die bezeichnend sind für eine Gruppe von einzelnen Okular-Antworten zu einem visuellen Stimulus; Software zum Bestimmen einer Verteilung visueller Resourcen zu jeder von mindestens zwei Zeitpunkten für jeden Teil von Individuen. Das System umfasst ferner Software zum Bestimmen und zur Quantifizierung einer Gruppen-Verteilung von visuellen Resourcen zu jedem Zeitpunkt und Software zum Erzeugen einer Anzeige von Gruppen-Verteilung visueller Resourcen zu dem visuellen Stimulus. Durch Vergleich der angezeigten Daten können Divergenzen und Konvergenzen detektiert werden.

Die bekannten Eye-Tracker liefern zeitaufgelöste Positionsdaten des Kopfes und der Augen einer Versuchsperson. Aus diesen Daten können die zeitaufgelösten Schnittpunkte der Sehstrahlen der Augen des Betrachters mit den Objekten einer statischen oder dynamischen Szene, wie sie z.B. bei der Betrachtung eines Plakates, eines Werbespots, der Bedienkonsole einer Maschine (z.B. Auto- oder Flugzeug-Cockpit), der Oberfläche eines Computers, etc. entstehen, ermittelt werden. Dazu werden die Positionsdaten des Kopfes und der Augen des Betrachters mit einer relativ hohen Abtast-Frequenz von bis zu 2000 Hz und mehr bestimmt.

Für die Aufzeichnung stehen üblicherweise verschiedene Techniken zur Verfügung. Die für eine breite Massenanwendung geeignetste Technik, da minimal invasiv und damit für die Versuchsperson nicht belastend oder einschränkend, besteht in der videobasierten Aufzeichnung des Kopfes der Versuchsperson unter zwei verschiedenen Winkeln im Infrarotbereich und einer sich anschließenden Bildverarbeitung, welche die Positionsdaten des Kopfes und der Augen aus den Bilddaten errechnet.

Aus der Bildanalyse können die Kopfposition und Iris- / Pupillenpositionen nach vorheriger Kalibrierung ermittelt werden. Diese oftmals in Echtzeit durchgeführte Ermittlung erfolgt durch die vom Hersteller des Eye-Trackers gelieferte Software. In dieser Form sind die Daten für den Endanwender, z.B. einen Psychologen, welcher das Betrachtungsschema eines Werbespots untersucht, jedoch nicht interpretierbar. Daher besteht der Bedarf, die Daten Endanwender-freundlich aufzubereiten.

Üblicherweise geschieht die Klassifizierung und Interpretation der Daten durch die Zerlegung der Verlaufsdaten in Fixierungen (Cluster von Blickpunkten) und Blicksprüngen (Sakkaden), wobei aus (Kognitions-) psychologischer Sicht die Fixierungen interessieren und die Sakkaden für grundlegende physiologische Prozesse interessant sind. Jedoch beruhen die hierfür bekannten Rechenverfahren bzw. Algorithmen auf einer Bewertung der Blickpunkte nach deren räumlicher Dispersion oder/und deren Geschwindigkeit bzw. Beschleunigung. Die Algorithmen erfordern eine Parametrisierung vom Endnutzer, bzw. Voreinstellungen für die Parameter vom Hersteller, welche einen möglichst großen Anwendungsbereich abdecken, dadurch aber für spezielle Anwendungsfälle zu ungenau sein können, da die Wahl der Parametrisierung der Algorithmen das Ergebnis signifikant beeinflussen kann. Ferner sind die Parameter zum Teil individuellen, also auf die Versuchsperson bzw. Personengruppe bezogenen, Schwankungen unterworfen oder/und sind von den im Versuch gestellten Aufgaben abhängig. Daher muss bei den bekannten Rechenverfahren bzw. Algorithmen der Endnutzer Erfahrung in der Wahl der Parameter mitbringen, was in der Praxis nur selten der Fall ist oder er muss sich auf eine ungefähre Parametrierung ("Daumenregel") verlassen. Die bekannten Rechenverfahren bzw. Algorithmen sind auch nicht auf die Betrachtung von dynamischen Szenen ausgelegt, in denen der Betrachter bewegten Objekten mit den Augen folgt. Die Abtrennung einer weiteren Art von Augen- und Blickbewegung, der sogenannten glatten Folgebewegung, wie sie bei der Betrachtung von dynamischen Szenen auftritt, gilt gegenwärtig als schwer und wurde bislang noch nicht zufriedenstellend gelöst.

Es ist daher Aufgabe der Erfindung, ein Verfahren und eine Vorrichtung vorzuschlagen, die eine Auswertung von Augen- und Blickverlaufsdaten ermöglichen, wobei die eingangs genannten Nachteile überwunden werden. Insbesondere soll die Erfindung das schnelle und zuverlässige Erkennen von Fixierungen und Sakkaden ermöglichen, um Steuerungen von Mensch-Maschine-Interaktionen zu verbessern. Außerdem soll die Erfindung das Erkennen von glatten Folgebewegung (engl. smooth pursuits) bei der Betrachtung dynamischer Szenen ermöglichen. Die Erfindung soll für weite Bereiche der Technik einsetzbar sein, nämlich überall dort, wo Mensch-Maschine-Interaktionen zur Steuerung von Maschinen, Geräten, Computern, Anlagen, Fahrzeugen usw. gewünscht oder erforderlich sind. Die Erfindung soll auch in wissenschaftlichen Gebieten einsetzbar sein, wie z.B. Ergonomie (engl. human factors), Lesen, Neurologie, Physiologie, Psychologie usw. Die Erfindung soll auch im Bereich des Marketings und der Werbung einsetzbar sein.

Zur Lösung der Aufgaben wird ein erfindungsgemäßes Verfahren mit den Merkmalen des Anspruchs 1 vorgeschlagen. Bevorzugte Verfahren gemäß der Erfindung werden in den abhängigen Ansprüchen 2-12 dargelegt.

Es wird also ein Verfahren bereitgestellt, welches eine ternäre Klassifizierung der Positionsdaten in Fixierungen und Sakkaden sowie (optional) glatte Folgebewegungen in robuster und in für die Untersuchung spezifischer Weise ermöglicht und welche vom Endanwender nicht parametrisiert werden müssen, bzw. eine intuitive, visuell geleitete Konfigurierung ermöglichen, wenn dies vom Anwender aus Gründen der Nachvollziehbarkeit und Validierung gewünscht ist.

Die Erfindung stellt eine integrierte Lösung zur Auswertung von Augen- und Blickbewegungen in der Form von Verlaufsdaten dar und verwendet Augen- und Blickverlaufsdaten, welche als zeitaufgelöste Ortsdaten beim Einsatz von Eye-Trackern in diversen Themengebieten entstehen, wie z.B. Usability-, Werbe- und Marktforschung, Computersteuerung (Mensch-Maschine-Schnittstelle), Fahr-/ Flugsimulatoren, Psychologie, Medizinische Diagnostik etc.. Das erfindungsgemäße Verfahren verwendet die Augen- und Blickverlaufsdaten in ihrer Rohform (Rohdaten) und wendet auf diese ein oder mehrere Verarbeitungsschritte an, um a) Rauschen/Datenfehler zu reduzieren und um b) aus den Rohdaten Fach-/ Themenspezifische Informationsinhalte zu extrahieren.

Die hier vorgeschlagene Lösung beinhaltet ein integriertes System von Verfahrensschritten zur automatisierten Durchführung von a) und b) und liefert eine Kodierung bzw. einen Code zur Darstellung des Blickprozesses und zur Auswertung desselben. Alle Verfahrensschritte der vorgeschlagenen Lösung bzw. die Rechenmodule werden in einer Vorrichtung zur Steuerung bzw. Unterstützung von Mensch-Maschine-Interaktionen integriert und ermöglichen es, dass die Steuerung vom Endnutzer in intuitiver Weise bedient werden kann.

Ein wesentlicher Aspekt des hier vorgestellten Verfahrens ist die Verdichtung bzw. Beschreibung der Daten durch die Kombination von Methoden aus einem weiten Spektrum der Mathematik (Geometrie, Topologie, Algebra, Differentialgleichungen, etc.) sowie die Interpretation der so beschriebenen Daten durch Informationstheoretische Methoden. Die Systematisierung der gewonnenen Beschreibungen erfolgt durch den Einsatz von Algorithmen des maschinellen Lernens. Durch die Kombination der Methoden wird der vom Endnutzer benötigte hohe Grad an Automatisation und Datenkondensation erreicht sowie der für die Interpretation der Ergebnisse nötige Grad an Abstraktion vermittelt. Die Verarbeitungsstrecke ist für den Endanwender parameterfrei, was besonders vorteilhaft ist, da der Endanwender normalerweise kein methodisches Wissen auf der Ebene der zur Anwendung kommenden Algorithmen mitbringt, sondern sich darauf verlässt, dass die implementierten Verfahren ihm die benötigten Endauswertungen in robuster Form zur Verfügung stellen. Die möglichst hohe Robustheit und Verlässlichkeit der erfindungsgemäßen Lösung ist insbesondere vorteilhaft zu sehen im Zusammenhang mit der Blicksteuerung von Maschinen und in der Medizinischen Diagnostik. Die Erfindung ist aber nicht auf diese Anwendungsbereiche beschränkt, sondern kann generell für jede Form von Mensch-Maschine-Interaktion benutzt werden.

Die Erfindung kann in einem breiten Spektrum von möglichen Einsatzbereichen zur Anwendung kommen: Gegenwärtig von besonderem Interesse ist der Bereich der sog. Gaze-Contingent Technologies bzw. Displays. Dieser Bereich umfasst die durch den Blick gesteuerte Anpassung des Computerbildschirms an die Interessensinhalte des Nutzers bzw. Users. Dazu wird der Blick des Nutzers durch zwei Kameras am Computer aufgezeichnet und der Interessensbereich des Nutzers auf dem Bildschirm berechnet. In Abhängigkeit von dem dort dargestellten Inhalt wird dann die lokale Information angepasst, so könnte z.B. eine Lesehilfe den Text dort vergrößern oder bei einer Stockung im Leseprozess von fremdsprachlichen Texten Übersetzungen anbieten. Für Smartphones ist eine selektive Vergrößerung (Zoomen) oftmals wünschenswert, da die Displaygröße limitiert ist.

Ein weiterer Einsatzbereich ist die Steuerung von Mouse-Zeigern, Cursorn und dergleichen. Für die Bedienung des Computers wird an einer "Blick-Mouse" gearbeitet, d.h. an einer durch den Blick des Benutzers gesteuerten Mouse-Zeiger-Bewegung (komplementär zum mit dem Touch Screen). Für die Unterstützung von Menschen mit einer schweren körperlichen Behinderungwird wird an der Umsetzung eines "Blick-Keyboards" gearbeitet, d.h. an einer durch den Blick des Benutzers gesteuerten Tastatur-Eingabe. An sich ist die Integration der Kameras in die Hardware eines Computers gegenwärtig kein technisches oder preisliches Problem mehr. Die momentane Restriktion besteht in der Auswertung und Interpretation der Blickdaten. So ist die Blickbewegung im Gegensatz zur Bewegung der Hände eine zum größten Teil unbewusste Aktion, deren gezielte Nutzung noch viele Fragen aufwirft. Dieses Beispiel lässt sich nahezu beliebig erweitern:

Beispielsweise dienen blickgesteuerte Fahrassistenzsysteme dazu, den Autofahrer in Abhängigkeit von der Verkehrssituation mit zusätzlicher Information gezielt zu versorgen bzw. ihn bei gefährlichen Situationen zu warnen (beginnende Ermüdung, kein Blick in den Rückspiegel bei Überholprozessen etc.). In der Medizin zeigt sich ein bedeutendes Potential für die Frühdiagnostik von neurologischen Erkrankungen, wenn große Patientengruppen systematisch auswertbar werden. In der Werbung gilt dies für die Untersuchung von Konsumentengruppen. Hier kommen mobile Gaze-Tracker zum Einsatz. Die Prototypen von Brillen mit integrierten Computern zeigen, dass die Restriktionen durch die Hardware immer kleiner werden.

Das hier vorgestellte Verfahren umfasst auch den Vergleich von mehreren Blickpfaden, um Ähnlichkeiten zwischen den Blickverhalten zu erkennen, zu qualifizieren und zu quantisieren, insbesondere in Form von Ähnlichkeitswerten bzw. einer Ähnlichkeitsmatrix, anhand derer eine Klassifizierung bzw. ein Clustering von Blickverhalten durchgeführt werden kann. Dieser Aspekt kann auch als eigenständige Lösung verstanden werden.

Hierzu werden für mindestens zwei Blickpfade jeweils eine Matrix erstellt wird und diese verarbeitet und einem Vergleich unterzogen werden, um für jeden Vergleich einen Ähnlichkeitswert zu erhalten, der die Ähnlichkeit der den mindestens zwei Blickpfaden zugrunde liegenden Blickverhalten anzeigt.

Dabei wird der Ähnlichkeitswert der Blickpfade durch deren Kolmogorov-Komplexität charakterisiert in Form ihrer Kompressibilität, welche auf einer Kombination von prädiktiver Kodierung, Lauflängen-Kodierung und Huffman-Kodierung der jeweils ersten Matrix basiert.

Vorzugsweise wird dazu die Matrix eines jeden Blickpfades in Untermatrizen zerlegt, deren Größe abhängig von der Abtastfrequenz der BlickerfassungsVorrichtung ist.

Dann werden die Untermatrizen jeweils einer harmonischen Analyse unterzogen, insbesondere einer Analyse mittels Walsh-Funktionen oder Cosinus-Funktionen, um transformierte Untermatrizen zu erhalten.

Anschließend werden die transformierten Untermatrizen einer jeden Matrix einem Kompression-Prozess unterzogen, um eine komprimierte Matrix zu erhalten, welche die am meisten signifikanten Koeffizienten enthält und ein reduziertes Volumen aufweist.

Dabei wird das reduzierte Volumen oder die Kompressibilität durch einen Kompressions-Wert repräsentiert, der die Länge in Bit der komprimierten Matrix des jeweiligen Blickpfades angibt.

Bevorzugt werden deutlich mehr als zwei Blickpfade, die durch ihre Matrizen repräsentiert werden, paarweise kombiniert, um für jede Kombination einen Ähnlichkeitswert zu erhalten, der die Ähnlichkeit der den mehreren Blickpfaden zugrunde liegenden Blickverhalten anzeigt. Dabei kann der Ähnlichkeitswert insbesondere gemäß der folgenden Gleichung ermittelt werden:

$$x = \frac{C(a,b) - \min\{C(a), C(b)\}}{\max\{C(a), C(b)\}} \quad (1)$$

wobei C(a) und C(b) die Länge in Bit der komprimierten Matrix des jeweiligen Blickpfades a bzw. b bezeichnen, und C(a,b) die Länge des kombinierten Blickpfades aus a und b bezeichnen.

Die berechneten Ähnlichkeitswerte bilden dann die Elemente einer Ähnlichkeitsmatrix, welche als Grundlage für einen Klassifizierungs- bzw. Clustering-Vorgang verwendet wird, wobei die Ähnlichkeitsmatrix insbesondere einem Klassifizierungs- bzw. Clustering-Vorgang unterzogen, der einen hierarchisch strukturierten Baum oder eine selbstorganisierte Karte erstellt.

Das Verfahren eignet sich besonders zur Blicksteuerung einer Mensch-Maschine-Interaktion, zur Diagnose mentaler Krankheiten oder psychischer Verwirrung, oder zum Forschen im Bereich Marketing und Werbung. Zahlreiche weitere Anwendungsgebiete sind denkbar.

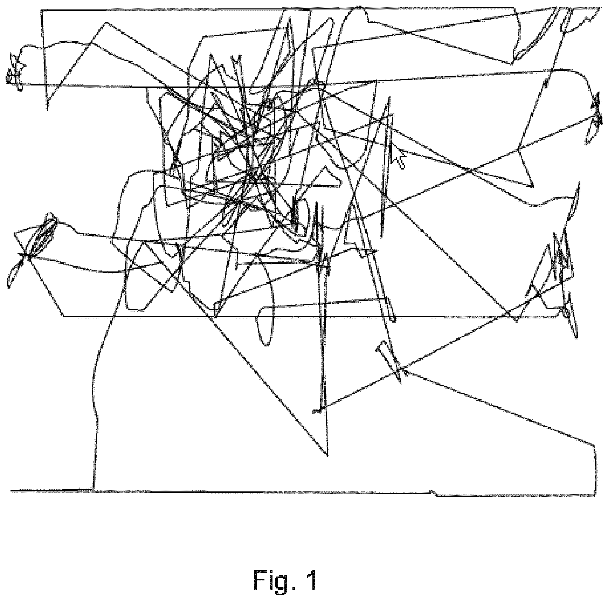

Die Erfindung wird nachfolgend anhand von Ausführungsbeispielen und unter Bezugnahme auf die beiliegenden Zeichnungen (Figuren 1-13) beschrieben:

In der Fig. 1 ist in üblicher Darstellung ein Sehstrahlendiagramm (engl. scanpath) gezeigt, welches den Blickverlauf einer Testperson beim Betrachten einer Bildvorlage (z.B. Internetseite) wiedergibt. Dort wo sich der Blickpfad für eine Zeitdauer lokal auf einen Bereich beschränkt, spricht man von einer Fixierung. Dort wo der Blickpfad schnell von einer Fixierung zur nächsten Fixierung verläuft, spricht man von Sakkaden. Das Sehstrahlendiagramm repräsentiert die grafische Darstellung von Augen- und Blickverlaufsdaten, welche ein herkömmlicher Eye-Tracker liefert. Für die Aufzeichnung der Daten stehen verschiedene Techniken zur Verfügung. Die für eine breite Massenanwendung geeignetste Technik, da minimal invasiv und damit für die Versuchsperson nicht belastend oder einschränkend, besteht in der videobasierten Aufzeichnung des Kopfes der Versuchsperson unter zwei verschiedenen Winkeln im Infrarotbereich und einer sich anschließenden Bildverarbeitung, welche die Positionsdaten des Kopfes und der Augen aus den Bilddaten errechnet. Die Erfindung setzt auf den Kopf-/ Augen-Positionsdaten, bzw. den Schnittpunktdaten der Sehstrahlen mit den betrachteten Objekten auf.

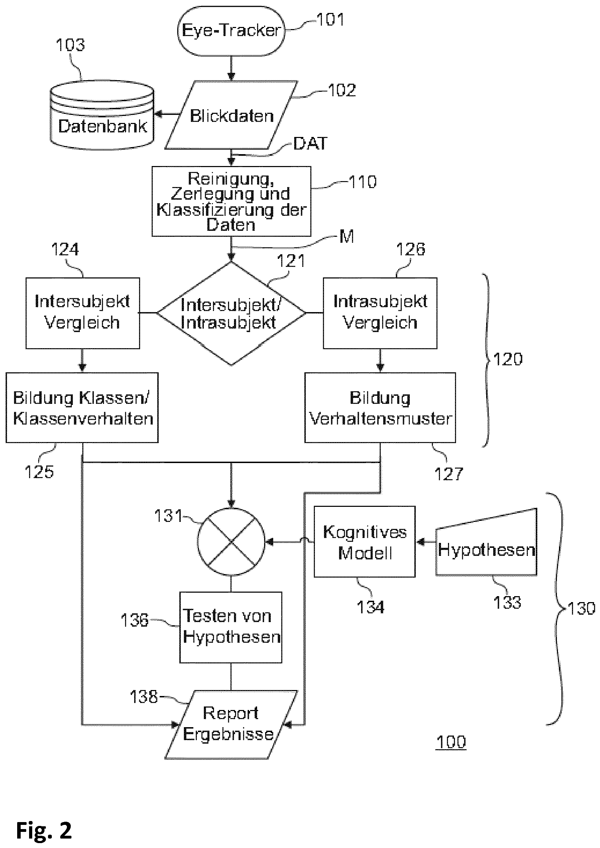

Die Fig. 2 zeigt ein Ablaufdiagramm für das erfindungsgemäße Verfahren 100, welches grob in folgende Abschnitte unterteilt werden kann:

a) Erfassen und Speichern der Bilddaten mittels Eye-Tracker (Schritte 101 - 103);
b) Erste Datenaufbereitung, insbesondere Reinigung, Zerlegung und Klassifizierung (Schritt bzw. Block 110), wobei aus den Rohdaten DAT eine erste Matrix M erzeugt wird;
c) Weitere Datenaufbereitung (optionaler Block 120), wobei in der erzeugten Matrix (bereinigte Daten) eine oder mehrere Strukturen erkannt werden, die für typische individuelle Verhaltensmuster (Schritte 126-127) oder gruppenspezifische Verhaltensweisen stehen (Schritte 121-125);
d) Nachfolgende Datenauswertung (Block 130), wobei die aufbereiteten Daten (Matrix) mit einem oder mehreren kognitiven Modellen verglichen wird, um das sich ausprägende Verhaltensmuster bzw. die Verhaltensweise einem typischen Verhalten bzw. einem diesem zugrunde liegenden kognitiven Prozess zuzuordnen.

Die Erfindung kann ein handelsübliches Gerät zur Aufzeichnung von Augen- und Blickverlaufsdaten (Eye-Tracker; Block 101) und eine Datenbank (Block 103) zur Speicherung der Daten verwenden. In der Datenbank kann auch eine Softwarebibliothek abgelegt sein, welche die Algorithmen zur automatischen Auswertung der Daten beinhaltet, sowie einer Arbeitsoberfläche und Repräsentationsschicht, welche die Module dem Endnutzer in einer intuitiven, seiner fachlichen Fragestellung gerechten Form zur Verfügung stellt.

**Die Erfindung ermöglicht eine schnelle und zuverlässige Zerlegung des Blickpfades in Fixierungen, Sakkaden und glatte Folgebewegungen:**

Denn es wird hier ein Verfahren bereit gestellt, welches eine ternäre Klassifizierung der Positionsdaten in Fixierungen, Sakkaden und glatte Folgebewegungen in robuster und in für die Untersuchung spezifischer Weise ermöglicht und welche vom Endanwender nicht parametrisiert werden müssen, bzw. eine intuitive, visuell geleitete Konfigurierung ermöglichen, wenn dies vom Anwender aus Gründen der Nachvollziehbarkeit und Validierung gewünscht ist. Außerdem erlaubt die Erfindung eine Anbindung der gewonnenen und aufbereiteten Daten an kognitive Architekturen (s. Block 130 in Fig. 2).

**Die Erfindung liefert eine Anbindung an kognitive Architekturen:**

Aufgrund der Komplexität der Prozesse, welche den Augen- und Blickbewegungen zugrunde liegen, existiert derzeit kein definitives Modell, was den empirischen Augen- und Blickverlauf erschöpfend beschreiben kann. Zum gegenwärtigen Stand der Forschung ist es vorherrschende Meinung, dass es sich, von einfachen Paradigmen abgesehen, um eine Mischung aus tiefliegenden physiologischen Basisprozessen (Bottom-Up) und höheren kognitiven Prozessen (Top-Down) handeln muss. Für die meisten in der Praxis relevanten Fragestellungen muss eine Modellierung beide Ebenen in Betracht ziehen. Um die höheren kognitiven Prozesse berücksichtigen zu können, stellt die vorgeschlagene Lösung eine Schnittstelle zu den gängigen kognitiven Architekturen bereit.

**Bevorzugte Ausführungen der Erfindung können auch die Bildung von Gruppen von Betrachtern durch Maschinelles Lernen umfassen:**

Hierzu wird eine Kombination von Auswertungsmethoden vorgeschlagen, welche einen Vergleich und eine Klassifizierung von Blickpfaden über Versuchsgruppen hinweg in robuster Weise ermöglicht. Die Auswertung erfordert dabei keinen spezifischen Input vom Endnutzer, sondern liefert die Daten in der vom Endnutzer benötigten abstrahierten Form. Dazu müssen die Rohdaten in eine Form gebracht werden, die einen expliziten Vergleich in der Form von Vergleichsmetriken ermöglicht. Die vorgeschlagene Lösung stellt derartige Metriken bereit und liefert die Klassifikation / Clusterung in der Form von Graphen. Desweiteren stellt die Lösung ebenfalls die geläufigen Metriken zur Verfügung, um zu etablierten Methoden kompatibel zu sein.

Eine verlässliche Bewertung des angenommenen Modells wird durch den expliziten Vergleich der Augen- und Blickverlaufsdaten möglichst umfangreicher Versuchsgruppen verbessert. Diese Vergleichsdaten können den gängigen statistischen Tests genügen. Die Lösung integriert gängige Teststatistiken, z.B. ANOVA, etc..

**Was die Datenspeicherung und Algorithmen-Implementierung angeht, so wird folgendes beachtet:**

Aufgrund der hohen Aufzeichnungsrate für die Daten (bis zu 2000 Hz und mehr), der zeitlichen Länge von praxisrelevanten Untersuchungen (ein Werbespot dauert einige zig Sekunden bis Minuten) sowie der Größe von Versuchsgruppen kommen sehr große Datenvolumina zustande. Deren effektive Verarbeitung erfordert selbst bei modernster Hardware entsprechend ausgebildete Verfahren, um die Arbeitslast für die Maschine und den Menschen möglichst gering zu halten. Die vorgeschlagene Lösung stellt dazu neuartige, im Zusammenhang mit Blickverlaufsdaten noch nicht verwendete, Verfahren bereit. Die Implementierung der Algorithmen nutzt die Möglichkeiten zum Rechnen auf Multiprozessor-Architekturen und dem Rechnen auf der / den Grafikkarte(n).

Ein weiterer wesentlicher Aspekt in der Umsetzung der integrierten Lösung ist der Laufzeitaspekt. Alle Auswertungen können interaktiv erfolgen und benötigen keine längeren Rechenzeiten auf einem handelsüblichen PC / Laptop. Dies ist eine für den Praxiseinsatz von großem Vorteil.

**Bevorzugte Ausführungen der Erfindung können auch eine Augen- / Blickverlaufsbeschreibung durch Raum-Zeit-Matrix vorschlagen:**

Die Protokollierung der Positionsdaten erfolgt als eine endliche Folge von Blickpunkten $GP_i$. Die Blickpunkte werden als eine Liste von Zahlentripeln $(t, x, y)_{i \in \{0,i,...,n\}}$ gespeichert. Die Menge der Zahlentripel $\{GP_i\}$ sei bezeichnet mit $GP = \{GP_i\}$, wobei die Koordinaten $x, y, t$ entweder kontinuierlich oder als diskret angenommen ("Pixelkoordinaten") werden, wobei die Messungen aufgrund der endlichen Messgenauigkeit immer diskret sind.

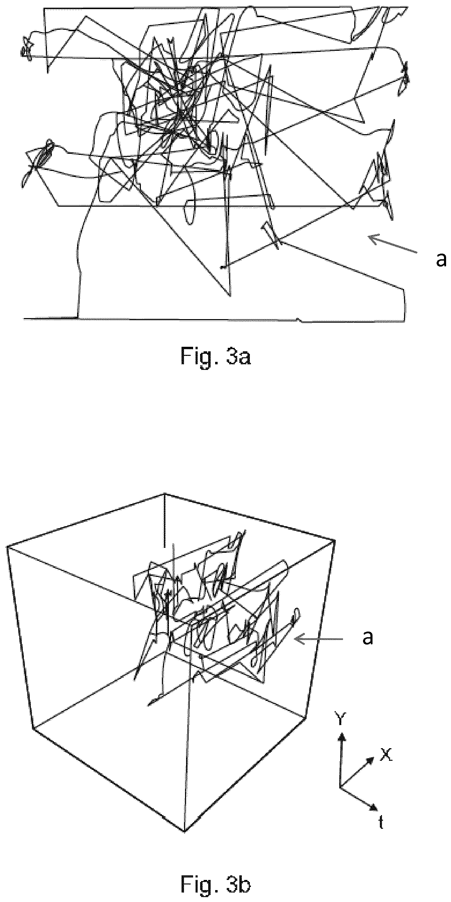

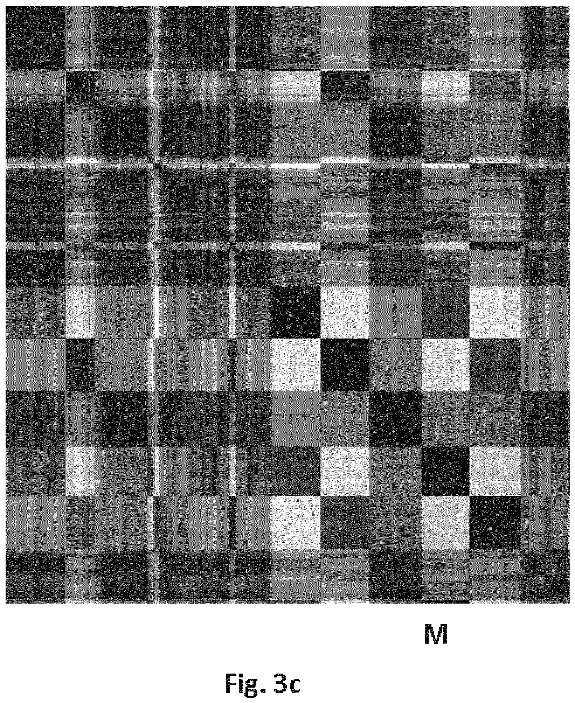

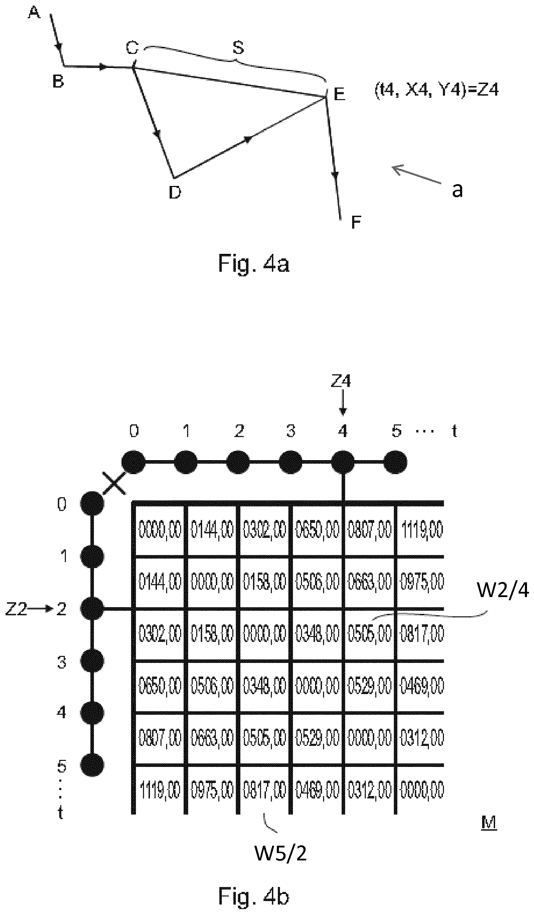

Die hier vorgeschlagene Lösung beschreibt die Daten durch eine Raum-Zeit-Matrix (Space-Time-Matrix, ST-Matrix, STM) $[d]_p$, welche sämtliche mögliche Distanzen d (siehe auch S in Fig. 4a) zwischen allen Raum-Zeit-Punkten beinhaltet. Die Distanz d zwischen zwei Raum-(Zeit)-Punkten kann bzgl. mehrerer Metriken (Euklidisch, Minkowski $I_p$, Minkowski Raum-Zeit) bestimmt werden. Dies führt zu einer bzgl. der jeweiligen Symmetriegruppe invarianten Darstellung. Um zu einem besseren intuitiven Verständnis zu gelangen, kann die Distanzmatrix als Graubild dargestellt werden. Das Graubild wird durch die (affine) Abbildung der nicht-negativen Distanzen in den Grauwertebereich erhalten. Dies sei exemplarisch für den oben gezeigten Pfad bzgl. der Euklidischen Metrik anhand der Figuren 3a-c gezeigt:

Die den Rohdaten, die durch das Sehstrahlendiagramm repräsentiert werden (Fig. 3a entspricht einer verkleinerten Darstellung der Fig. 1) können auch in einer dreidimensionalen Darstellung mittels Raum-Zeit-Punkten dargestellt werden (siehe Fig. 3b mit den Raumkoordinaten x, y und der Zeitkoordinate t). Die Matrix entspricht einer Kodierung bzw. eine Informations-Code für das Blickverhalten (engl. visual behavior). Durch die nachfolgend beschriebene Datenverarbeitung (Block 110 in Fig. 2) wird die Information weiter komprimiert , woraus sich eine hoch-effiziente und aussagekräftige Darstellung desBlickverhaltens ergibt.

Die Matrix M ist symmetrisch, da die (Euklidische) Distanz symmetrisch ist. Deutlich in der Matrix zu erkennen, ist eine blockartige/ rektanguloide/ rektilineare/ isothetische Struktur, welche die Clusterung des Pfades in der Raum-Zeit darstellt. Diese Struktur ist die Grundlage zur Extraktion der Pfadabschnitte aus den Daten. Das isothetische Grundmuster bzgl. der Zeitachse macht die STM einfach auswertbar.

Anhand der Fig. 4a und 4b wird die Berechnung bzw. Bildung der Matrix näher veranschaulicht. Die Fig. 4a zeigt einen sehr vereinfachten Sehstrahlenverlauf (engl. gaze trajectory) ausgehend vom Blickpunkt A bis zum Blickpunkt E. Die Fig. 4a zeigt zum einfacheren Verständnis nur den Anfang eines Blickverlaufs und nicht einen kompletten Blickverlauf wie z.B. in Fig. 1. Jeder Blickpunkt wird durch ein Zahlentripel repräsentiert, das die Zeit t, und die jeweiligen Raumkoordinaten X und Y angibt. Der Blickpunkt E hat beispielsweise das Zahlentripel (t4, x4, y4), welches auch als Zahlengruppe Z4 verstanden werden kann. Erfindungsgemäß werden nun die Zahlengruppen Z0, Z1, Z2, Z3, Z4, ...Zn paarweise miteinander kombiniert, wobei jeweils die Distanz S (siehe Fig. 4a) zwischen den zwei Bildpunkten ermittelt und in eine Matrix M (s. Fig. 4b) eingetragen wird.

In der Fig. 4b ist der Aufbau der Matrix M veranschaulicht. Der Wert W2/4 gibt die Distanz zwischen den Blickpunkten E und C bzw. Zahlengruppen Z2 und Z4 an. Die Operation wird für alle möglichen Kombination durchgeführt. Bei einer Abtastfrequenz von 1000 Hz ergibt sich innerhalb eines Messzeitraumes von 1 Sekunde bereits ein Datenvolumen vom 1000x1000 Zahlentripeln. Dieses Datenvolumen wird aber durch die später beschriebenen Filterungen deutlich reduziert.

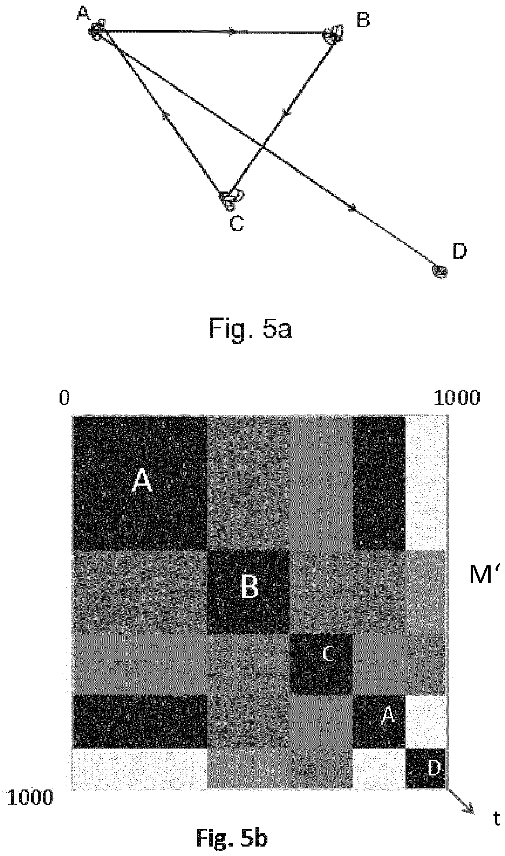

Zunächst erhält man eine recht umfangreiche erste Matrix. Dies wird anhand eines weiteren Beispiels (Fig. 5a und 5b) veranschaulicht:

Wie dort in Fig. 5a gezeigt, ist der Blickverlauf etwas umfangreicher als in Fig. 4a, wobei der Blick zunächst vom Bereich A (erste Fixierung) ausgeht und zum Bereich B (zweite Fixierung) springt (erste Sakkade). Dann geht es zum Bereich C (dritte Fixierung) und von dort zurück zum Bereich A (vierte Fixierung) und dann zum Bereich D. Es wird also die erste Fixierung A nochmals aufgesucht, d.h. die Daten der ersten Fixierung korrelieren stark mit den Daten der vierten Fixierung.

Dieses Blickverhalten (engl. visual behavior) wird nicht vollkommen durch das Blickverlaufs-Diagramm der Fig. 5a (oder Fig. 4a) wiedergegeben, weil bei dieser Darstellung nur die räumliche Dimension gezeigt werden kann und nicht auch die zeitliche Dimension. Allerding wird das Blickverhalten vollkommen in der Struktur der erhaltenen Matrix M' nach Fig. 5 wiedergegeben (ebenso durch M in Fig. 4b):

Ausgehend von t=0 (linke obere Ecke) bis zu t= 1000 (rechte untere Ecke) zeigt sich zunächst ein größeres dunkles Quadrat, dessen Ausdehnung die zeitliche Verweildauer am Fixierungsbereich A wiedergibt. Dann folgt (entlang der Diagonalen) ein etwas kleines dunkles Quadrat, dessen Ausdehnung die zeitliche Verweildauer am Fixierungsbereich B wiedergibt. Die benachbarten Flächen (Kopplungsfelder) zeigen durch ihren Grauwert an, dass dieses zweite Quadrat einen anderen Fixierungsbereich (nämlich B) angibt, der zu dem ersten Bereich A beabstandet ist. Die Grauwerte sind also ein Maß für die Distanz zwischen den Bereichen A, B, C .... Wie anhand des vorletzten Quadrats zu erkennen ist, gibt es zwei sehr dunkle Flächen, die diesem und dem ersten Quadrat zugeordnet sind (dieselbe Spalte bzw. Reihe). Der hohe Grauwert zeigt an, dass die beiden Quadrate sehr wenig voneinander beabstandete Bereichen betreffen: Das vorletzte Quadrat (auf der Diagonalen) betrifft nämlich auch den Bereich A (siehe Rücksprung in Fig. 5a). Das letzte Quadrat hat mit dem ersten Quadrat jedoch nur fast weiße Kopplungsfelder gemein, d.h. das letzte Quadrat betrifft einen Bereich (nämlich D), der sehr weit vom ersten Bereich A entfernt ist.

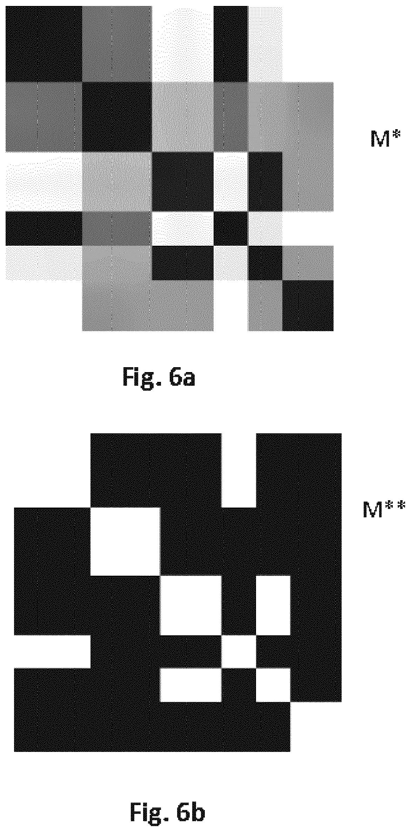

Das Datenvolumen der Matrix M bzw. M' kann deutlich verringert werden, ohne dass die Aussagefähigkeit der Matrix-Struktur darunter leidet. Im Gegenteil: Durch die hier vorgeschlagene Datenreduzierung (Filterung, Schwellwert) wird die Prägnanz der Matrixstruktur noch weiter erhöht (gefilterte Matrix in Fig. 6a) und kann sogar in eine standardisierte Darstellung überführt werden (Scharz-Weiss-Matrix quasi wie 2D-Matrixcode in Fig. 6b), um mit üblichen Darstellungen ("ABC-String") in Einklang gebracht werden zu können.

**Bevorzugte Ausführungen der Erfindung können auch eine Extraktion der Pfadabschnitte durch Raum-Zeit-Filterung umfassen:**

Eine grundlegende Beschreibung des Blickpfades (engl. scanpath), wie es gegenwärtiger Stand ist, erfolgt durch die symbolische Angabe der Fixierungen als eine Buchstabenfolge "ABCADEBE..." sowie die den Fixierungen zugeordneten Größen - Dauer einer Fixation/ Ausmaß einer Fixation. Desweiteren können Größen zur Charakterisierung einer Sakkade bestimmt werden - Sprungweite einer Sakkade / Richtung einer Sakkade. Die gängigen Lösungen erreichen die Extraktion dieser Größen durch die Ableitung der Sakkaden aus den Pfaddaten gemäß der eingangs genannten Kriterien. Hierzu ist oftmals eine händische Datenauswertung erforderlich. Die Fixierungen werden dann als die Abschnitte zwischen den Sakkaden gesetzt.

Die vorgeschlagene Lösung extrahiert die interessierenden Größen dagegen aus der Raum-Zeit-Matrix und kann voll-automatisch durchgeführt werden. Die Extraktion erfolgt in mehreren Schritten. Der erste Schritt besteht in einer Filterung der Raum-Zeit (Space-Time-Filtering *STF*). Dies ist eine anisotrope Filterung mit zwei gekoppelten Kernen, z.B. zwei Gauß-Kernen:

$$STF([d]_p) = \frac{1}{W_p} \sum_{q \in T^2} G_{\sigma_t}(\|p - q\|) G_{\sigma_s}(|d_p - d_q|) d_q$$

Der Faktor Wp normalisiert die Gauß-Gewichte zu 1:

$$W_p = \sum_{q \in S} G_{\sigma_t}(\|p - q\|) G_{\sigma_s}(|d_p - d_q|)$$

Die Parameter $\sigma_t$ und $\sigma_s$ sind dabei primär nicht als numerischen Parameter zu betrachten, sondern als Parameter mit grundlegender physiologischer Relevanz und hängen damit vom physiologischen / psychologischen Modell ab. Ein kleines $\sigma_t$ bedeutet eine enge zeitliche Kohärenz im Prozess der Betrachtung und ein kleines $\sigma_s$ bedeutet eine enge räumliche Kohärenz zwischen den betrachteten Inhalten. Die Vorgehensweise der Filterung ist analog der bilateralen Bildverarbeitung. Das Ergebnis ist ein reduziertes Schema, welches die Grundinformationen zum Prozess auf Ebene der Augen- / Blickdaten in zweidimensionaler Form enthält. Ein solches Schema zeigt die geglättete (engl. smooth filtered) Matrix M* in Figur 6a.

Die quadratischen Blöcke entlang der Hauptdiagonalen der Matrix (s. auch Fig. 5b) kodieren die Fixierungen. Die Kantenlänge, bzw. die Anzahl der Zellen entlang der Diagonalen eines Blocks, ist die Dauer der Fixierung. Die Grauwerte in den quadratischen Blöcken entlang der Diagonalen der Raum-Zeit-gefilterten STM (durch Glätten bzw. anisotropes Filtern erhaltende Matrix M*) entsprechen dabei der mittleren räumlichen Ausdehnung der Fixation. Die Grauwerte in den rechteckigen Blöcken im restlichen Teil der Matrix entsprechen den mittleren Abständen zwischen den Fixierungen. Durch das Setzen eines Schwellenwertes für den räumlichen Abstand in der STM auf die räumliche Dispersion einer mittleren Fixierung kann die Matrix weiter vereinfacht werden, woraus sich eine Schwellwert-gefilterte Matrix M** ergibt. Dies wird anhand der Fig. 6b im Vergleich mit Fig. 6a veranschaulicht. Demnach wird aus der ersten Matrix (s. M in Fig. 1 oder M' in Fig. 5b) eine erste Glättungs-gefilterte Matirx M* erzeugt, aus der wiederum eine dritte Schwellwert-gefilterte Matrix M** erzeugt werden kann. Diese Matrix M** kann auch direkt aus der ersten (Rohdaten-) Matrix M bzw. M' erzeugt werden.

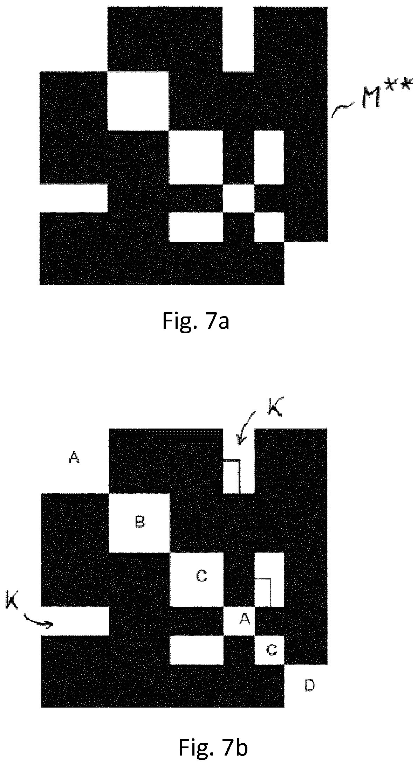

In dieser Darstellung entspricht die äußere Form der Codierung des Blickpfades der bekannten Matrix Code Kodierung, wie sie z.B. bei DataMatrix zur Kodierung von Internet-Links, Postwertzeichen, Waren, etc. Verwendung findet. Ersetzt man jetzt die Quadrate der Diagonalen durch Buchstaben und berücksichtigt dabei die Kopplungen, so erhält man die zum gegenwärtigen Zeitpunkt übliche Darstellung des Blickpfades durch eine Buchstabenfolge (Stringdarstellung) "ABCACD". Dies wird anhand der Figuren 7a und 7b veranschaulicht.

Die Darstellung des Blickpfades als Buchstabenfolge ("ABC-String") ist der momentane Standard. Die Ableitung zeigt aber auch, wie viel Information auf den Weg zur Stringdarstellung verworfen wurde.

Die Bereitstellung / Berücksichtigung dieser Information ist ebenfalls Gegenstand der Erfindung. Dazu schlägt die Erfindung die Codierung des Blickpfades in der Form eines erweiterten Matrix Codes gemäß der Figur 5b vor. Der Matrix Code ist erweitert, weil er keine schwarzen/weißen Module sondern Module mit Grauwerten nutzt, Matrix-Grauwerte-Code. Diese Darstellung verschlüsselt wesentlich mehr Information als die Stringdarstellung und ist die Grundlage erweiterter Auswertungen. Zu Zwecken der Vergleichbarkeit kann die vorgeschlagene Lösung aber auch mit der Stringdarstellung arbeiten. So werden die Levenshtein-Distanzen zwischen Strings bestimmt.

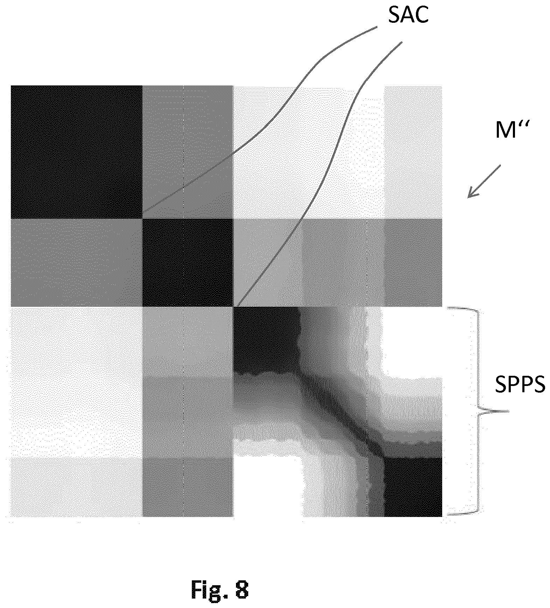

Die anisotrope Filterung lässt sich als der Grenzfall einer anisotropen Diffusion auffassen, welche durch eine Partielle Differentialgleichung beschrieben wird. Durch die geeignete Wahl des Diffusionstensors lassen sich weitere gesuchte Aspekte aus der STM extrahieren. Dies ermöglicht insbesondere, die glatte Folgebewegung sauber aus den Daten zu extrahieren. Die glatte Folgebewegung (engl. smooth pusuit) ist bei dynamischen Szenen relevant, wo der Betrachter mit den Augen einem bewegten Objekt folgt. Für die automatische Bestimmung der glatten Folgebewegung gibt es zurzeit noch keinen Standard. Die simultane ternäre Bestimmung von Sakkaden, Fixierungen und glatten Folgebewegungen für dynamische Szenen gilt als außergewöhnlich kompliziert. Die Anwendung einer anisotropen Diffusion auf die Space-Time-Matrix STM lässt diesen Abschnitt im Blickpfad neben den Fixierungen und Sakkaden einfach erkennen. In der Fig. 8 ist beispielhaft eine Matrix M" dargestellt, in welcher der Bereich der glatten Folgebewegung SPPS eine Linien- bzw. Gradienten-Struktur zeigt im Gegensatz zu den Sakkaden SAC, die scharfe Kanten bzw. Ecken aufweisen. Die Matrix M'' kann als vierte Matrix verstanden werden, welche die Anwendung einer anisotropen Diffusion aus der ersten Matrix (siehe z.B. Matrix M' in Fig. 5b) gewonnen wird.

**Die Erfindung umfasst auch einen Vergleich von Blickpfaden:**

Für die weitergehenden Auswertungen ist die (informationstheoretische) Struktur der Distanzmatrix maßgeblich (s. auch Block 120 in Fig. 2). Grundlegend für das weitere Vorgehen ist die Informationsäquivalenz zwischen der Distanzmatrix und dem Spektrum der Distanzen. Das heißt formal, dass die Verteilung der Distanzen (Spektrum der Distanzen) $his_{GP}(d) \equiv \#\{(i,j)|0 \le i < j \le n, d(GP_i, GP_j) = d\}$ $his_{GP}(d) = \#\{(i,j)|0 \le i < j \le n, d(GP_i, GP_j) = d\}$ im wesentlichen nur einen Blickpfad als Lösung besitzt. Damit lassen sich zwei Blickpfade bzw. zwei unterschiedliche Ausschnitte aus einem Blickpfad, durch einen Vergleich der jeweiligen Histogramme in Beziehung setzen. Als Ähnlichkeitsmaß dienen die üblichen Distanzmaße für Spektren, wie z.B. die Kullback-Leibler Divergenz, Jensen Shannon Divergenz, $X^2$-Tests, Kolmogorov-Smirnov-Test, Earth-Mover's-Distance, Histogramm-Durchschnitt, Kosinus-Distanz, Euklidische-Distanz, etc.

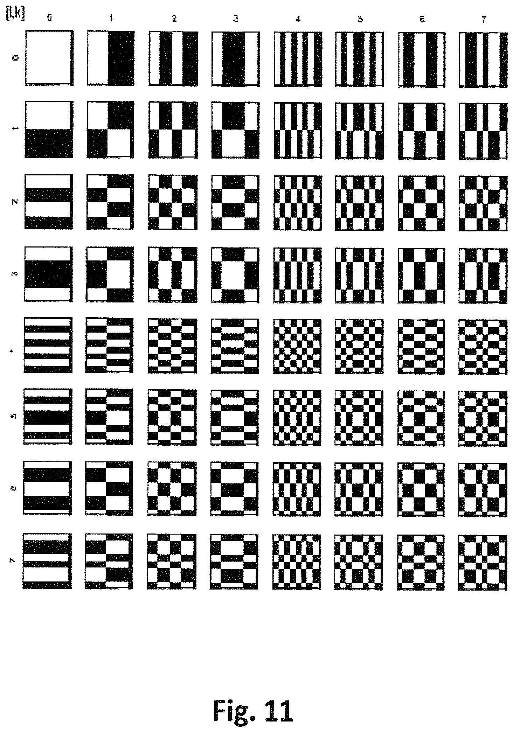

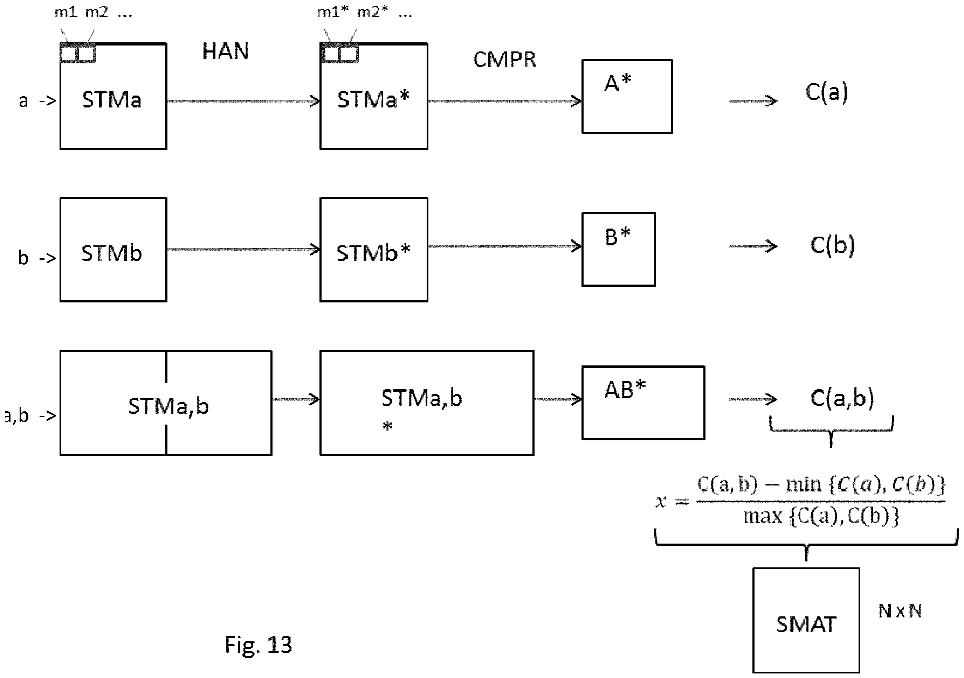

Eine Steigerung oder Reduktion in der Vergleichstiefe lässt sich erreichen, wenn die übergeordnete Struktur der Distanzmatrix betrachtet bzw. nicht betrachtet wird. Dazu wird die Struktur der Distanzmatrix als Textur aufgefasst. Ausgewertet wird das Tamura-Textur-Histogramm, Gabor-Features, Local-Image-Feaure-Patches-Histogramm, Local-Image-Feature-SIFT-Histogramm, Edge-Histogramm, ... Bevorzugte Ausführungen der Erfindung stellen weitere Varianten zum Vergleich von Blickpfaden bereit. Dies wird hier unter Bezugnahme auf die Figuren 11-13 beschrieben. In der Fig. 13 werden die Verarbeitung/Aufbereitung und der Vergleich von zwei Blickpfaden veranschaulicht, die durch zwei Matrizen STMa und STMb repräsentiert werden.

Eine Variante beruht auf der normalisierten Kompressionsdistanz der in der Form von STMs kodierten Blickpfade.

$$x = \frac{C(a, b) - \min\{C(a), C(b)\}}{\max\{C(a), C(b)\}} \quad (1)$$

C(a) und C(b) bezeichnen die Länge in Bit der komprimierten STMs (Raum-Zeit-Matrizen) der Pfade a und b, C(a,b) bezeichnet die Länge des kombinierten Pfades aus a und b. Die Komprimierung der Pfade erfolgt dabei mit den standardmäßigen Verfahren der Informatik zur Datenkomprimierung, jedoch in einer auf die Problemstellung zugeschnittenen Weise. Die Komprimierung der jeweiligen STM (siehe STMa, STMb sowie kombinierte STMa,b in Fig. 13) stellt die Approximation an die Kolmogorov-Komplexität der jeweiligen STM dar, welche definiert ist, als die Länge des kürzesten Programms zur Berechnung der STM auf einem universellen Computer. Dies sei im Folgenden an zwei Beispielen erläutert. Es sei festgehalten, dass die Wahl des Komprimierungsverfahrens prinzipiell beliebig sein kann. Insbesondere ermöglicht aber die der Struktur der STM angepasste Wahl die Fokussierung auf die aus fachlicher Sicht interessierenden Aspekte und verkürzt die Rechenzeit auf praktikable Zeiten. Die Erfindung macht sich deshalb die an sich bekannten gängigen Verfahren der Informatik zur Datenkomprimierung zu Nutze und ermöglicht dem Endnutzer eine variable Vorgehensweise. Die Nutzung der Kolmogorov-Komplexität als theoretische Grundlage zur Datenanalyse ist in der Literatur bekannt und wird deshalb nicht weiter ausgeführt. Für die Erfindung maßgeblich ist deren konkrete Implementation in der Kombination mit der STM:

Die erfindungsgemäße Bestimmung dieser speziellen Distanz geschieht in zwei generellen Schritten (s. auch Fig. 13): In einem ersten Schritt wird die STM (z.B. STMa) in Untermatrizen (m1, m2, ...) zerlegt, wobei deren Größe variabel ist. Diese richtet sich nach der Samplingrate des Eye-Trackers und der für die Fragestellung sinnvollen physiologischen Zeitskala (wenn Fixationen betrachtet werden, liegt diese im Bereich von 100 ms). So könnte eine 128x128 Matrix in 16x16 8x8 Matrizen zerlegt werden. Eine Potenz von 2 ist nicht erforderlich, vereinfacht aber die Skalierung. Die einzelnen Untermatrizen werden anschließend einer diskreten harmonischen Analyse unterzogen (s. HAN in Fig. 13). Dazu kommen mehrere Funktionen-Systeme zum Einsatz. Die Wahl des Systems hängt von der Detailtiefe der STM ab. So sind Walsh-Funktionen für die gefilterte Schwarz-Weiß-Matrix besonders geeignet. In der beiliegenden Fig. 11 werden beispielhaft die Walsh-Funktionen für das 8x8-System gezeigt.

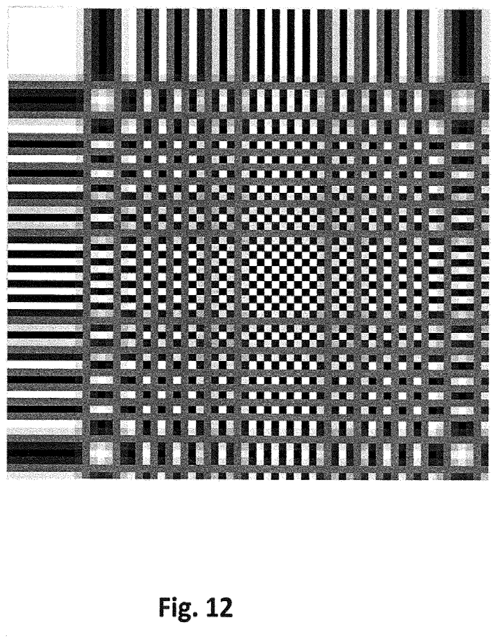

Ein für die Darstellung im Grauwertbereich geeignetes System besteht aus den diskreten Cosinus-Funktionen. In der Fig. 12 werden hierzu ebenfalls die für ein 8x8 System genutzten Cosinus-Funktionen gezeigt.

Die Ergebnisse der harmonischen Analyse HAN, die auf jede Untermatrix m1, m2, ... einer jeden STM angewendet wird (d.h. auf die Untermatrizen von STMa, STMb und STMa,b), sind transformierte Untermatrizen m1*, m2* ... des verwendeten Basis-Systems (z.B. Walsh). Dies bedeutet, dass jede Untermatrix (z.B. m1*) die Gewichte der jeweiligen Walsh-Funktion repräsentiert. Der erste Eintrag oder das bedeutendsten Gewicht (engl. most significant weight) in den Untermatrizen ist prädiktions-kodiert.

Der zweite Schritt beinhaltet den eigentlichen Vergleich durch Kompression CMPR. Dieser beruht auf einer Entropie-Kodierung der transformierten Pfade in separater und in kombinierter Fassung. Die Transformations-Gewichte werden dazu entweder blockweise kodiert oder in für jede Basisfunktion separaten Sequenzen kodiert. Die Entropie-Kodierung erfolgt z.B. in der Form einer Huffman-Kodierung oder einer arithmetischen Kodierung. Da es nur auf den relativen Vergleich der Kompressionsrate ankommt, dient die arithmetische Kodierung der theoretischen Untersuchung des Verfahrens. Die komprimierten Matrizen A*, B* und AB* enthalten im Wesentlichen die bedeutendsten Koeffizienten (engl. most significant coefficients) und weisen dadurch ein (deutlich) reduziertes Volumen gegenüber den Eingangs-Matrizen auf. Die Kompressionsrate (etwa 10-40%) bzw. das verringerte Volumen kann durch die Werte C(a), C(b) und (Ca,b) ausgedrückt werden. Dies bedeutet, dass in der finalen Form drei kodierte Blickpfade vorliegen, die gemäß obiger Gleichung (1) zu einem Distanzausdruck bzw. Ähnlichkeitswert verbunden werden. Dies geschieht für alle zu vergleichenden Pfadpaare. Das Ergebnis ist eine Koeffizienten-Matrix (siehe Ähnlichkeits-Matrix SMAT in Fig. 13), welche als Input für Clusterverfahren dient. Das Clustering erfolgt mit den gängigen Verfahren aus dem Datamining und liefert z.B. einen hierarchisch gegliederten Baum oder eine Self-organizing map.

Die beschriebenen Vergleiche sind wesentlich exakter und tiefliegender als die momentan in der Literatur beschriebenen Verfahren. Diese repräsentieren die Folge der Fixierungen in einer Zeichenkette und nutzen dann die String-Editing Distanz und dynamische Programmierung als Maß für die Ähnlichkeit bzw. Unähnlichkeit. Die Bestimmung des Strings erfolgt dabei durch die Zuordnung der im ersten Schritt bestimmten Fixierungen auf die im Stimulus / Szene vorhandenen Areas-of-Interest (AOI) oder auch Regions-of-Interest (ROI). Die Bestimmung der AOI kann dabei durch eine einfache Gitterung des Raumes / der Ebene oder durch vom Endnutzer nach menschlichen Ermessen bestimmten Kriterien erfolgen (Gridded AOI und Semantic AOI). Eine weitere Aggregation kann durch die Clusterung der Fixierungen durch Clusteralgorithmen erfolgen.

**Bevorzugte Ausführungen der Erfindung können auch die Darstellung der Blickverlaufsdaten und die Formulierung einer Hypothese zum Blickpfad umfassen:**

Die erhobenen Blickverlaufsdaten im Zusammenhang mit der dargebotenen Szene erlauben eine Bestimmung des Netzhautbildes des Betrachters. Dies ermöglicht es, die so bestimmte Szene in der Form einer 3D-Darstellung dem Auswerter zur Verfügung zu stellen. Der Auswerter sitzt gewissermaßen im Auge der Versuchsperson. Dadurch kann der Auswerter ein genaues subjektives Bild darüber gewinnen, was die Versuchsperson betrachtet hat. Dies erlaubt es, genauer eine Hypothese über den Blickverlauf im Zusammenhang mit der Szene zu formulieren. Die Darstellung der Szene kann dabei in 3D erfolgen, wobei der 3D Effekt durch Farbverschiebung als auch durch den Einsatz einer Shutter-Technologie erreicht wird. Wahlweise kann auch die 2D Darstellung erfolgen.

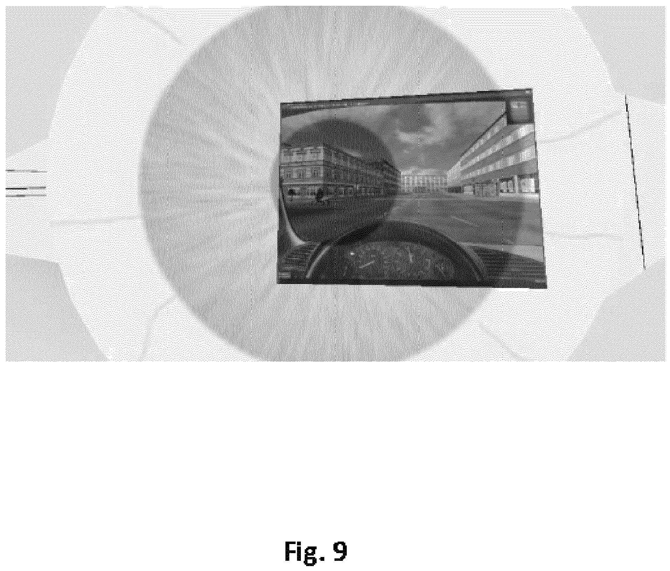

Diese sei exemplarisch anhand der Figur 9 gezeigt, da eine 3D-Darstellung nicht in gedruckter Form (auf Druckpapier etc.) möglich ist.

**Zum Formulieren einer Hypothese zum Blickpfad ist folgendes zu sagen:**

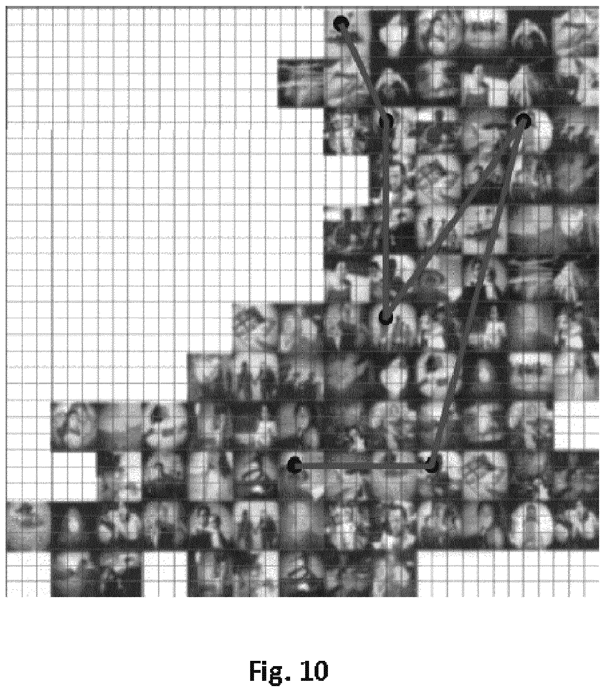

Die betrachtete Szene kann räumlich quantisiert werden. Die Raumbereiche entsprechen dabei den ROIs. Mit der Maus kann durch Tasteneinzeldruck die Fixation in einer ROI simuliert werden. Die Anzahl der Tastendrücke entspricht der Dauer der Fixation. Diese ist als Parameter frei wählbar. Der so gewonnene hypothetische Blickpfad wird protokolliert und kann danach mit den Versuchsdaten der Einzelpersonen in der Datenbank verglichen werden. Die statistische Auswertung der Ähnlichkeiten zwischen den Hypothesen und den Beobachtungsdaten gibt dann die nötige Sicherheit zu Wahrscheinlichkeit der formulierten Hypothese. Durch dieses Vorgehen kann auf hoher abstrakter Ebene ein Prozessmodel für die Augen- / Blickdaten gewonnen werden und direkt an den experimentellen Daten getestet werden. Siehe Figur 10.

**Die Erfindung ermöglicht auch den Export des Blickprozesses in einen kognitiven Prozess:**

Die Interpretation des so gewonnenen Prozessmodells für die Augen- / Blickdaten geschieht in einem Kognitiven Modell (s. auch Block 130 in Fig. 2). Es existieren innerhalb der Psychologie mehrartige Kognitive Modelle / Architekturen, z.B. ACT, SOAR, EPIC, etc. Die vorgeschlagene Erfindung stellt eine Schnittstelle bereit, um das Prozessmodell für die Augen- Blickdaten an das Prozess-Modell für den kognitiven Prozess anzubinden.

**Zudem können bevorzugte Ausführungen der Erfindung eine integrierte Datenspeicherung umfassen:**

Aufgrund der weitreichenden fachlichen Fragestellungen ist das Sammeln und der Austausch von Daten durch multizentrische Studien wünschenswert. Dies erfordert eine möglichst offene und einfach strukturierte Datenorganisation.

Die Lösung beinhaltet zwei Formen der Datenspeicherung. Für die Verwaltung umfangreicher Datensätze steht eine Anbindung an eine marktgängige relationale SQL-Datenbank zur Verfügung. Weiterhin können die Daten in XML-formatierter Weise in Textdateien direkt gespeichert werden (NoSQL Konzept). Während der Laufzeit des Programes arbeitet die Lösung aufgrund der Performance mit einer In-Memory-Datenbank. Bei der Anlegung der einzelnen Datensätze vergibt die Lösung GUIDs, um bei multizentrischen Studien die Mehrfachvergabe von Schlüsseln zu vermeiden und damit die Zusammenführung von mehreren Datensätzen zu ermöglichen.

**Zusammenfassend können folgende Eigenschaften hier kurz festgehalten werden:**

Die Erfindung kann in bevorzugten Ausführungen ein Verfahren zur Erhebung, Sammlung, Darstellung und/oder Auswertung von Blickverlaufsdaten in integrierter Umgebung liefern. Die Darstellung von Augen- / Blickbewegungen erfolgt über einen Matrix-Grauwerte-Code.

Die Erfindung kann in bevorzugten Ausführungen eine simultane ternäre Zerlegung von Augen - bzw. Blickbewegungen in Fixierungen, Sakkaden und glatten Folgebewegungen bei der Betrachtung von statischen und dynamischen Szenen leisten. Es wird ein Vergleich von Blickpfaden durch Vergleiche von Histogrammen für verschiedene Features ermöglicht.

Das hier vorgeschlagene Verfahren dient u.a. zur Erhebung von Blickverlaufsdaten für vorgegebene Paradigmen, wie Freie Betrachtung, Search Task, Raven's progressive Matrizen, Stroop, Change Blindness, etc.. Das Verfahren dient auch zur Erhebung von frei vorgegebenen statischen und dynamischen Szenen, wie der Betrachten eines Werbeplakates, Werbespots, Films, Videospiels, Flug-/Fahrsimulation, Interaktion mit einer grafischen Benutzeroberfläche. Es ermöglicht die manuelle Definition von ROIs über statischen und dynamischen Szenen. Auch ermöglicht das Verfahren eine automatische Bestimmung von statischen und dynamischen ROIs.

Das Verfahren ist zur Speicherung von Blickverlaufsdaten für multizentrische Studien im Zusammenhang mit den Daten der Blickparadigmen geeignet. Auch ist das Verfahren zur automatischen Segmentierung des untersuchten Kollektivs in Ähnlichkeitscluster geeignet. Es wird u.a. eine statistische und visuelle Darstellung der Befunde in integrierter Umgebung erzielt.

Das Verfahren eignet sich zur 3D Darstellung der Blickdaten / Rückrechnung der Daten auf das Sehfeld und dessen Darstellung als 3D Szene. Auch eignet sich das Verfahren zur freien Definition einer Hypothese zum Blickverlauf und Vergleich der Hypothese mit den Daten der Datenbank sowie statistische Tests zur Hypothese.

Diese Beschreibung beschreibt mehrere Ausführungsformen. Die beschriebenen Ausführungsformen sind jedoch nicht so zu verstehen, dass sie notwendigerweise die Erfindung definieren. Die beschriebenen Ausführungsformen definieren nur dann die Erfindung, wenn sie in den Umfang der beigefügten Ansprüche fallen. Daher ist der Schutzumfang der vorliegenden Erfindung ausschließlich durch die beigefügten Ansprüche definiert.

**Patentansprüche**

1. Verfahren zur Kodierung von Augen- und Blickverlaufsdaten (DAT) für die Blicksteuerung einer Mensch-Maschine-Interaktion in einer technischen Vorrichtung, bei dem die Augen- und Blickverlaufsdaten (DAT) Zeit- und Raumparameter (t, x, y) als zeitaufgelöste Ortsdaten in Form von Zahlengruppen (Z0, Z1, Z2...) angeben, die mittels einer Blickerfassungsvorrichtung (ET) gewonnen werden und jeweils einem Blickpunkt (A, B, C, D, E ...) innerhalb eines Blickpfades (a) zugeordnet werden, wobei aus den Zahlengruppen jeweils zwei Zahlengruppen (ZO,Z1; Z0,Z2; ZO,Z3...) miteinander kombiniert werden, um für jede Kombination einen Wert (W2/4) zu erhalten, der zumindest den räumlichen Abstand (S) zwischen den zwei Blickpunkten (E, C) angibt, denen die jeweiligen Zahlengruppen (Z2, Z4) zugeordnet sind, wobei die erhaltenen Werte (W2/4) eine Kodierung für die Augen- und Blickverlaufsdaten (DAT) darstellen, **dadurch gekennzeichnet, dass** die Werte (W2/4) in Form einer ersten Matrix (M), die den Blickpfad (a) repräsentiert, erfasst und gespeichert werden, und dass für mindestens zwei Blickpfade (a, b) jeweils eine Matrix (STMa, STMb) erstellt wird und diese verarbeitet und einem Vergleich unterzogen werden, um für jeden Vergleich einen Ähnlichkeitswert (X) zu erhalten, der die Ähnlichkeit der den mindestens zwei Blickpfaden (a, b) zugrunde liegenden Blickverhalten anzeigt, und dass der Ähnlichkeitswert (X) der Blickpfade (a, b) durch deren Kolmogorov-Komplexität charakterisiert wird in Form ihrer Kompressibilität (C), welche auf einer während der Verarbeitung der für die Blickpfade (a, b) erstellten Matrizen (STMa, SZMb) durchgeführten Kombination von prädiktiver Kodierung, Lauflängen-Kodierung und Huffman-Kodierung der jeweiligen Matrix (STMa, STMb) basiert, um diese auf ihre bedeutendsten Koeffizienten zu komprimieren, und wobei die erhaltenen Werte (W2/4) und Ähnlichkeitswerte (X) für die Mensch-Maschine-Interaktion in der technischen Vorrichtung genutzt werden.

2. Verfahren nach Anspruch 1, wobei die Zahlengruppen (Z0, Z1, Z2...) entsprechend der Zeit- und Raumparameter (t, x, y) Zahlentripel (t0, x0, y0; t1, x1, y1; ...) darstellen.

3. Verfahren nach Anspruch 1, wobei die erste Matrix (M, M') einer Glättungs-Filterung, insbesondere einer anisotropen Filterung, unterzogen wird, um eine zweite Matrix (M*) zu erhalten, die gegenüber der ersten Matrix (M) einen reduzierten Datenumfang aufweist.

4. Verfahren nach Anspruch 3, wobei die erste oder zweite Matrix (M; M*) einer Schwellwert-Filterung unterzogen wird, um eine dritte Matrix (M**) zu erhalten, die gegenüber der ersten und zweiten Matrix (M*) einen reduzierten Datenumfang aufweist.

5. Verfahren nach Anspruch 1 oder 3, wobei die erste Matrix (M') einer Glättungs-Filterung in Form einer anisotropen Diffusion unterzogen wird, um eine vierte Matrix (M") zu erhalten, die gegenüber der ersten Matrix (M') einen reduzierten Datenumfang aufweist.

6. Verfahren nach einem der Ansprüche 1-5, wobei die erste, zweite, dritte und/oder vierte Matrix eine Kodierung für Fixierungen und Sakkaden des Blickpfades (a) darstellt.

7. Verfahren nach Anspruch 1, wobei die Matrix (STMa, STMb) eines jeden Blickpfades (a, b) in Untermatrizen (m1, m2, ...) zerlegt wird, deren Größe abhängig von der Abtastfrequenz der BlickerfassungsVorrichtung (ET) ist.

8. Verfahren nach Anspruch 7, wobei die Untermatrizen (m1, m2, ..) jeweils einer harmonischen Analyse (HAN) unterzogen werden, insbesondere einer Analyse mittels Walsh-Funktionen oder Cosinus-Funktionen, um transformierte Untermatrizen (m1*, m2*, ..) zu erhalten.

9. Verfahren nach Anspruch 8, wobei die transformierten Untermatrizen (m1*, m2*, ...) einer jeden Matrix (STMa) einem Kompression-Prozess (CMPR) unterzogen werden, um eine komprimierte Matrix (A*) zu erhalten, welche die am meisten signifikanten Koeffizienten enthält und ein reduziertes Volumen aufweist.

10. Verfahren nach Anspruch 9, wobei das reduzierte Volumen oder die Kompressibilität durch einen Kompressions-Wert (C(a),...) repräsentiert wird, der die Länge in Bit der komprimierten Matrix (A*,...) des jeweiligen Blickpfades (a, ...) angibt.

11. Verfahren nach einem der Ansprüche 1-10, wobei mehrere Blickpfade (a, b, ...); die durch ihre Matrizen (STMa, STMb, ...) repräsentiert werden, paarweise kombiniert werden, um für jede Kombination einen Ähnlichkeitswert (X) zu erhalten, der die Ähnlichkeit der den mehreren Blickpfaden zugrunde liegenden Blickverhalten anzeigt, wobei der Ähnlichkeitswert (X) insbesondere gemäß der folgenden Gleichung ermittelt wird:

$$x = \frac{C(a, b) - \min\{C(a), C(b)\}}{\max\{C(a), C(b)\}} \quad (1)$$

wobei C(a) und C(b) die Länge in Bit der komprimierten Matrix (A*, B*) des jeweiligen Blickpfades a oder b bezeichnen, und C(a,b) die Länge des kombinierten Blickpfades aus a und b bezeichnen.

12. Verfahren nach Anspruch 11, wobei die Ähnlichkeitswerte (X) die Elemente einer Ähnlichkeitsmatrix (SMAT) bilden, welche als Grundlage für einen Klassifizierungs-Vorgang verwendet wird, wobei die Ähnlichkeitsmatrix (SMAT) insbesondere einem Klassifizierungs-Vorgang unterzogen wird, der einen hierarchisch strukturierten Baum oder eine selbst-organisierte Karte erstellt.

**Claims**

1. A method for encoding eye movements and eye tracking data (DAT) for gaze contingent control within a man-machine interaction in a technical device, in which the eye movements and eye tracking data (DAT) represent time and space parameters (t, x, y) as time-resolved location data in the form of groups of numbers (Z0, Z1, Z2 ...) which are obtained by means of an eye tracking device (ET) and are each assigned to a viewpoint (A, B, C, D, E ...) within a scanpath (a), two groups of numbers (Z0, Z1; Z0,Z2; Z0,Z3...) are combined with each other to obtain, for each combination, a value (W2/4) indicating at least the spatial distance (S) between the two viewpoints (E, C) to which the respective groups of numbers (Z2, Z4) are assigned, the obtained values (W2/4) representing an encoding for the eye movements and eye tracking data (DAT), **characterized in that** the values (W2/4) are determined and stored in the form of a first matrix (M) representing the scanpath (a), and **in that** for at least two scanpaths (a, b) a respective matrix (STMa, STMb) is created and processed and subjected to a comparison in order to obtain for each comparison a similarity value (X) which indicates the similarity of the visual behaviour underlying the at least two scanpaths (a, b), and **in that** the similarity value (X) of the scanpaths (a) is determined from the similarity value (X) of the scanpaths (a, b), b) **characterized by** their Kolmogorov complexity in the form of their compressibility (C) based on a combination of predictive, run-length, and Huffman encoding of the respective matrix (STMa, STMb), performed during the processing of matrices (STMa, SZMb) created for the scanpaths (a, b), to compress them to their most significant coefficients, and wherein the obtained values (W2/4) and similarity values (X) are used for man-machine interaction in the technical device.

2. The method according to claim 1, wherein the groups of numbers (Z0, Z1, Z2...) represent number triples (t0, x0, y0; t1, x1, y1; ...) corresponding to the time and space parameters (t, x, y).

3. The method according to claim 1, wherein the first matrix (M, M') is subjected to a smoothing filtering, in particular an anisotropic filtering, in order to obtain a second matrix (M*) which has a reduced data volume compared with the first matrix (M).

4. The method according to claim 3, wherein the first or second matrix (M; M*) is subjected to threshold filtering to obtain a third matrix (M**) having a reduced data volume compared to the first and second matrix (M*).

5. The method according to claim 1 or 3, wherein the first matrix (M') is subjected to a smoothing filtering in the form of an anisotropic diffusion to obtain a fourth matrix (M'') having a reduced data volume compared to the first matrix (M').

6. The method according to any of claims 1-5, wherein the first, second, third and/or fourth matrix represents an encoding for fixations and saccades of the scanpath (a).

7. The method according to claim 1, wherein the matrix (STMa, STMb) of each scanpath (a, b) is divided into sub-matrices (m1, m2, ...), the size of which depends on the scanning frequency of the eye tracking device (ET).

8. The method according to claim 7, in which the sub-matrices (m1, m2, ..) are each subjected to a harmonic analysis (HAN), in particular an analysis using Walsh functions or cosine functions, in order to obtain transformed sub-matrices (m1*, m2*, ..).

9. The method according to claim 8, wherein the transformed sub-matrices (m1*, m2*, ...) of each matrix (STMa) are subjected to a compression process (CMPR) to obtain a compressed matrix (A*) containing the most significant coefficients and having a reduced volume.

10. The method according to claim 9, wherein the reduced volume or compressibility is represented by a compression value (C(a), ...) indicating the length in bits of the compressed matrix (A*, ...) of the respective scanpath (a, ...).

11. A method according to any one of claims 1-10, wherein a plurality of scanpaths (a, b, ...) represented by their matrices (STMa, STMb, ...) are combined in pairs to obtain for each combination a similarity value (X) indicative of the similarity of the visual behaviour underlying the plurality of scanpaths, the similarity value (X) being determined in particular according to the following equation:

$$x = \frac{C(a, b) - \min\{C(a), C(b)\}}{\max\{C(a), C(b)\}} \quad (1)$$

where C(a) and C(b) denote the length in bits of the compressed matrix (A*, B*) of the respective scanpath a or b, and C(a,b) denotes the length of the combined scanpath of a and b.

12. The method according to claim 11, wherein the similarity values (X) form the elements of a similarity matrix (SMAT) which is used as a basis for a classification process, wherein the similarity matrix (SMAT) is in particular subjected to a classification process which creates a hierarchically structured tree or a self-organized map.

**Revendications**

1. Un procédé de codage des mouvements oculaires et des données de suivi oculaire (DAT) pour un contrôle contingent du regard au sein d'une interaction homme-machine dans un dispositif technique, dans lequel les mouvements oculaires et les données de suivi oculaires (DAT) représentent des paramètres spatio-temporels (t, x, y) comme des données d'emplacement à résolution temporelle résolues sous la forme de groupes de nombres (Z0, Z1, Z2 ...) qui sont obtenus au moyen d'un dispositif de détection du regard (ET) et qui sont attribués à un point de vue (A, B, C, D, E ...) dans trajet visuel (a), deux groupes de nombre (Z0, Z1 ; Z0, Z2 ; Z0, Z3 ...) étant combinés les uns avec les autres afin d'obtenir, pour chaque combinaison, une valeur (W2/4) indiquant au moins la distance spatiale (S) entre les deux points de vue (E, C) auxquels les groupes de nombres respectifs (Z2, Z4) sont affectés, les valeurs obtenues (W2/4) représentant un code pour les mouvement oculaires et les données de suivi oculaire (DAT), **caractérisé en ce que** les valeurs (W2/4) sont déterminées et stockées sous la forme d'une première matrice (M) représentant le trajet visuel (a), et **en ce que** pour au moins deux trajets visuels (a, b), est crée une matrice respective (STMa, STMb) qui est traitée et soumise à une comparaison afin d'obtenir pour chaque comparaison une valeur de similarité (X) qui indique la similarité du comportement visuel sous-jacents aux deux trajets visuels (a, b) au moins, et **en ce que** la valeur de similarité (X) des trajets visuels (a, b), **caractérisée par** leur complexité de Komogorov dans la forme de leur compressibilité (C) sur la base d'une combinaison d'un codage de prédictif, de longueur de course, et Huffman de la matrice respective (STMa, STMb), effectuée durant le traitement des matrices (STMa, SZMb) créées pour les trajets visuels (a, b), pour les compresser vers leurs coefficients les plus significatifs, et dans lequel les valeurs obtenues (W2/4) et les valeurs de similarité (X) sont utilisées pour une interaction homme-machine dans le dispositif technique.

2. Le procédé selon la revendication 1, dans lequel les groupes de nombres (Z0, Z1, Z2 ...) représentent des triplets de nombres (t0, x0, y0; t1, x1, y1, ...) correspondant aux paramètres spatio-temporels (t, x, y).

3. Le procédé selon la revendication 1 dans lequel la première matrice (M, M') est soumise à un filtrage de lissage, en particulier un filtrage anisotrope, afin d'obtenir une deuxième matrice (M*) ayant un volume de données réduit par rapport à la première matrice (M).

4. Le procédé selon la revendication 3, dans lequel la première ou la deuxième matrice (M; M*) est soumise à un filtrage de seuil pour obtenir une troisième matrice (M**) qui est une matrice à taille de données réduite par rapport aux première et deuxième matrices (M*).

5. Le procédé selon la revendication 3 ou 4, dans lequel la première matrice (M') est soumise à un filtrage de lissage sous forme de diffusion anisotrope pour obtenir une quatrième matrice (M") ayant une taille de données réduite par rapport à la première matrice (M').

6. Le procédé selon l'une des revendications 1-5, dans lequel les première, deuxième, troisième et/ou quatrième matrices représentent un codage pour des fixations et des saccades du trajet visuel (a).

7. Le procédé selon la revendication 1, dans lequel la matrice (STMa, STMb) de chaque trajet visuel (a, b) est décomposée en sous-matrices (m1, m2, ...) dont la taille dépend de la fréquence d'échantillonnage du dispositif de détection de regard (ET).

8. Le procédé selon la revendication 7, dans lequel les sous-matrices (m1, m2, ..) sont chacune soumises à une analyse harmonique (HAN), en particulier à l'aide de fonctions de Walsh ou de fonctions cosinus, pour obtenir des sous-matrices transformées (m1*, m2*, .. ).

9. Le procédé selon la revendication 8, dans lequel les sous-matrices transformées (m1*, m2*, ...) de chaque matrice (STMa) sont soumises à un processus de compression (CMPR) pour obtenir une matrice comprimée (A*) comportant les coefficients les plus significatifs et ayant un volume réduit.

10. Le procédé selon la revendication 9, dans lequel le volume réduit ou la compressibilité est représenté par une valeur de compression (C(a), ...) indicative de la longueur de bits de la matrice compressée (A* ,. ) du trajet visuel respectif (a, ...).

11. Le procédé selon l'une quelconque des revendications 1-10, dans lequel une pluralité de trajets visuels (a, b, ...) représentés par leurs matrices (STMa, STMb, ...) sont combinés par paires pour donner pour chaque combinaison une valeur de similarité (X) indicative de la similarité du comportement du regard sous-jacent aux multiples trajets visuels, la valeur de similarité (X) étant déterminée notamment selon l'équation suivante:

$$x = \frac{\mathrm{C(a,b)} - \min\left\{C(a), C(b)\right\}}{\max\left\{\mathrm{C(a)}, \mathrm{C(b)}\right\}} \quad (1)$$

où C(a) et C(b) désignent respectivement la longueur en bits de la matrice compressée (A*, B*) du trajet visuel respectif a ou b, et C(a, b) désigne la longueur du trajet visuel combiné de a et b.

12. Le procédé selon la revendication 11, dans lequel les valeurs de similarité (X) forment les éléments d'une matrice de similarité (SMAT) qui sert de base pour un procédé de classification, dans lequel la matrice de similarité (SMAT) est en particulier soumise à un procédé de classification créant un arbre structuré hiérarchiquement ou une carte auto-organisée.

Fig. 1

**Fig. 2**

Fig. 3a

Fig. 3b

M

Fig. 3c

A

B

C

S

E

(t4, X4, Y4)=Z4

D

F

a

Fig. 4a

Z4

0 1 2 3 4 5 ⋯ t

0

1

Z2 → 2

3

4

5
⋮
t

| 0000,00 | 0144,00 | 0302,00 | 0650,00 | 0807,00 | 1119,00 |
| 0144,00 | 0000,00 | 0158,00 | 0506,00 | 0663,00 | 0975,00 |
| 0302,00 | 0158,00 | 0000,00 | 0348,00 | 0505,00 | 0817,00 |
| 0650,00 | 0506,00 | 0348,00 | 0000,00 | 0529,00 | 0469,00 |
| 0807,00 | 0663,00 | 0505,00 | 0529,00 | 0000,00 | 0312,00 |
| 1119,00 | 0975,00 | 0817,00 | 0469,00 | 0312,00 | 0000,00 |

W2/4

W5/2

M

Fig. 4b

**Fig. 5a**

**Fig. 5b**

M*

Fig. 6a

M**

Fig. 6b

Fig. 7a

Fig. 7b

Fig. 8

**Fig. 9**

Fig. 10

Fig. 11

Fig. 12

Fig. 13

$$x = \frac{C(a, b) - \min\{C(a), C(b)\}}{\max\{C(a), C(b)\}}$$

EP 2 679 147 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9918842 A1
- US RE40014 E1
- WO 2009153724 A1
- US 2009086165 A1
- US 7029121 B2
- US 2010118030 A1
- US 2006189886 A1